# NEW EUROPEAN PATENT SPECIFICATION

(11) **EP 3 035 909 B2**
(45) Date of publication and mention of the opposition decision: **10.06.2020**
(45) Mention of the grant of the patent: 30.08.2017
(21) Application number: 14747004.1
(22) Date of filing: 30.07.2014
(51) Int. Cl.: A61K 8/49, A61Q 5/00, A61K 8/73, A61K 8/23, A61K 8/27

(54) **HAIR TREATMENT COMPOSITIONS**
HAARBEHANDLUNGSZUSAMMENSETZUNGEN
COMPOSITIONS DE TRAITEMENT CAPILLAIRE

(30) Priority: 21.08.2013 EP 13181182
(43) Date of publication of application: 29.06.2016
(73) Proprietor: Unilever PLC, a company registered in England and Wales under company no. 41424, London EC4Y 0DY (GB); Unilever N.V., 3013 AL Rotterdam (NL)
(72) Inventor: POLIDANO, Aurore Anabelle, Hull HU2 0AT (GB); ZDRAVKOVA, Aneliya Nikolova, Wirral Merseyside CH63 3JW (GB)
(74) Representative: Potter Clarkson
(86) International application number: PCT/EP2014/066433
(87) International publication number: WO 2015/024752

(56) References cited:
- WO-A1-2014/139757
- WO-A2-2013/010706
- US-A- 5 104 645
- US-A- 5 798 121
- US-A1- 2004 202 636
- US-A1- 2010 215 775
- US-A1- 2011 305 778

## Description

The invention relates to an anti-dandruff shampoo composition with improved colour stability.

Dandruff is an issue that affects many people globally. The condition is manifested by the shedding of clumps of dead skin cells from the scalp. These are white in colour and provide an aesthetically displeasing appearance. A factor that contributes to dandruff are certain members of the *Malassezia* yeasts. To combat these, antidandruff products have included certain anti-dandruff actives. One such antidandruff active is selenium disulfide.

A problem with selenium disulfide hair treatment compositions is that they are not colour stable. The colour of the formulation changes over time to an unattractive dark green colour, which is undesirable for consumers.

US 4,854,333 discloses that one way of overcoming the problem of selenium sulphide discolouration is to include peroxy oxidising agents, such as hydrogen peroxide or sodium percarbonate.

Alternative method of overcoming this discolouration without using peroxy oxidising agents are required.

It is an object of the invention to solve this problem.

We have found that by incorporation of zinc salt in the shampoo, the resulting composition is colour stable and doesn't turn green over time.

### Summary of the Invention

The invention thus provides in a first aspect an antidandruff shampoo according to claim 1.

Preferably the selenium disulfide is present at a level of from 0.1 to 1.5 wt.%, more preferably from 0.1 to 1 wt. %, most preferably 0.1 to 0.5 wt.%.

Preferably the zinc pyrithione is present at a level of from 0.1 to 3 wt.%, more preferably from 0.1 to 2 wt.%.

Preferably the cationic guar hydroxypropyltrimonium chloride polymer is present at a level of 0.01 to 5 wt.%, preferably from 0.05 to 1 wt.%, more preferably from 0.05 to 0.5 wt.%.

Preferably the antidandruff shampoo comprises from 0.5 to 20 wt.% alkyl sulfate and/or ethoxylated alkyl sulfate anionic surfactant.

Preferably the antidandruff shampoo comprises from 0.1 to 10 wt.% of a betaine surfactant, preferably an alkyl amidopropyl betaine.

Preferably the antidandruff shampoo comprises:-
a) from 0.1 to 2 wt.% of selenium disulfide;
b) from 0.1 to 5 wt.% of zinc pyrithione;
c) from 0.01 to 10 wt.% of a cationic guar hydroxypropyltrimonium chloride polymer;
d) from 0.5 to 20 wt.% alkyl sulfate and/or ethoxylated alkyl sulfate anionic surfactant;
e) from 0.1 to 10 wt.% of cocamidopropyl betaine; and
f) from 0.01 to 10 wt. % of a silicone.

Preferably the antidandruff shampoo comprises 0.05 to 5 wt.%, preferably from 0.1 to 2.5 wit.%, more preferably from 0.25 to 1 wt.% of a crosslinked polyacrylate polymer.

Preferably the antidandruff shampoo comprises from 0.1 to 8 wt.%, more preferably from 0.3 to 5 wt.% of a silicone.

A second aspect of the invention relates to the composition according to the first aspect of the invention for use in the treatment of dandruff.

### Detailed Description of the Invention

### Selenium Disulfide

Selenium disulfide (or selenium sulfide) are catch-all terms for the inorganic compound of approximate formula SeS₂. As sulphur and selenium form rings and chains readily, the selenium sulphide used in anti-dandruff treatment compositions is a mixture where the overall Se:S ratio is 1:2 (so is often abbreviated as SeS₂).

The selenium disulfide is present at a level of from 0.1 to 2 wt.%, preferably from 0.1 to 1.5 wt.%, more preferably from 0.1 to 1 wt.%, or even 0.1 to 0.5 wt.%.

### Zinc pyrithione (ZPT)

The hair treatment composition comprises a zinc pyrithione (ZPT) which is an alternate name for zinc 1-hydroxy-2-pyridinethione.

The zinc pyrithione is present at a level of from 0.1 to 5 wt.%, preferably from 0.1 to 3 wt.%, more preferably from 0.1 to 2 wt.% based on the total weight of the composition.

### Cationic Polymer

The hair treatment composition comprises a cationic guar hydroxypropyltrimonium chloride polymer.

These cationic polymers are commercially available from Rhone-Poulenc in their JAGUAR trademark series. Examples are JAGUAR C13S, which has a low degree of substitution of the cationic groups and high viscosity; JAGUAR C15, having a moderate degree of substitution and a low viscosity; JAGUAR C17 (high degree of substitution, high viscosity); JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

The cationic conditioning polymer will generally be present in compositions of the invention at levels of from 0.01 to 10 wt.%, preferably from 0.01 to 5 wt.%, preferably from 0.05 to 1 wt.%, more preferably from 0.05 to 0.5 wt.% based on total weight of the composition.

### Other anti-dandruff actives

Additional anti-dandruff actives may be included in the compositions. Illustrative substances are octopirox (piroctone olamine), azole antimicrobials (e.g. climbazole), and combinations thereof. Amounts of these materials may range from about 0.01 to about 5 wt.%, preferably from 0.1 to 3 wt.%, and optimally from about 0.3 to about 4 wt.% of the composition.

### Hair Treatment Compositions

### Shampoo Compositions

The invention is in the form of an anti-dandruff shampoo composition.

Shampoo compositions of the invention are preferably aqueous, i.e. they have water or an aqueous solution or a lyotropic liquid crystalline phase as their major component. Suitably, the composition will comprise from 50 to 98%, preferably from 60 to 90% water by weight based on the total weight of the composition.

### Surfactant

The anti-dandruff shampoo comprises a surfactant that acts to cleanse the head and scalp.

Preferably the surfactant comprises an anionic surfactant.

The anionic surfactant in total is present at a level of from 0.5 to 45 wt.%, more preferably from 1.5 to 35 wt.%, most preferred from 5 to 20 wt.%, based on the total weight of the composition.

Preferred anionic surfactants are an alkyl sulfate and/or ethoxylated alkyl sulfate anionic surfactant. Preferred levels of alkyl sulfate and/or ethoxylated alkyl sulfate anionic surfactant are from 0.5 to 20 wt.%.

Preferred alkyl sulfates are C₈₋₁₈ alky sulfates, more preferably C₁₂₋₁₈ alkyl sulfates, preferably in the form of a salt with a solubilising cation such as sodium, potassium, ammonium or substituted ammonium. Examples are sodium lauryl sulfate (SLS) or sodium dodecyl sulfate (SDS).

Preferred alkyl ether sulfates are those having the formula: RO(CH₂CH₂O)nSO₃M; wherein R is an alkyl or alkenyl having from 8 to 18 (preferably 12 to 18) carbon atoms; n is a number having an average value of greater than at least 0.5, preferably between 1 and 3; and M is a solubilising cation such as sodium, potassium, ammonium or substituted ammonium. An example is sodium lauryl ether sulfate (SLES).

A preferred ethoxylated alkyl sulfate anionic surfactant is sodium lauryl ether sulfate (SLES) having an average degree of ethoxylation of from 0.5 to 3, preferably 1 to 3.

Shampoo compositions according to the invention may comprise one or more further anionic cleansing surfactants which are cosmetically acceptable and suitable for topical application to the hair.

Examples of further suitable anionic cleansing surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable.

The composition can include co-surfactants, to help impart aesthetic, physical or cleansing properties to the composition.

An example of a co-surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco monoisopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more; preferably, the value of n lies from about 1.1 to about 2; most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92/06154 and US 5,194, 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.1 to about 10 wt.%, preferably from 0.5 to 8, more preferably from 1 to 5 wt.%, based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

A further optional but preferred surfactant is an alkyl glycinate and/or alkyl carboxyglycinate. If present, it is present at a level of from 1 to 8 wt.%, preferably 2 to 6 wt.%

Preferably the alkyl glycinate and/or alkyl carboxyglycinate has an alkyl group of from C₈₋₂₂ carbon atoms, in the form of a salt with a solubilising cation such as sodium, potassium, ammonium or substituted ammonium. Preferred glycinates are sodium coco glycinate and sodium cocoyl glycinate.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (inclusive of any co-surfactant) in a shampoo composition of the invention is generally from 1 to 50 wt.%, preferably from 2 to 40 wt.%, more preferably from 10 to 25 wt.% by total weight surfactant based on the total weight of the composition.

### Silicone

Advantageously compositions herein include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Number average particle size diameters for the silicones may range from about 0.01 micron to about 50 micron, most preferably from about 0.01 to about 0.5 micron.

Advantageously the compositions of this invention may include a pre-mix of a silicone microemulsion. The microemulsion is an aqueous surfactant stabilized emulsion of silicone particles having a number average particle diameter ranging from about 10 to about 1,000 nm, preferably from about 100 to about 500 nm. Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions or microemulsions of dimethiconol. Also suitable are amodimethicone emulsions such as DC939 (from Dow Corning) and SME253 (from GE Silicones).

Amounts of the silicone in compositions range from about 0.01 to about 10 wt.%, preferably from about 0.1 to about 8 wt.%, more preferably from about 0.3 to about 5 wt.% by weight of the shampoo compositions.

### Suspending Agent

Preferably the compositions of the invention further comprise a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493.

Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent, if included, will generally be present in a shampoo composition of the invention at levels of from 0.05 to 5 wt.%, preferably from 0.1 to 2.5 wt.%, more preferably from 0.25 to 1 wt.%.

The preferred suspending agent is a crosslinked polyacrylate polymer.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The Examples will now be illustrated with reference to the following non-limiting Examples. Inventions according to the invention are demonstrated by a number, comparative inventions are demonstrated by a letter.

### Examples

The invention will be illustrated by the following examples showing the advantages of the invention.

### Example 1 - SeS₂ is colour unstable and turns dark green

A base shampoo was formulated with the following ingredients:-

**Table 1 - Base Shampoo with SeS₂**

| **INCI name** | **Tradename** | **A (wt.%)** | **B (wt.%)** | **C (wt.%)** | **E (wt.%)** |
|---|---|---|---|---|---|
| Sodium Laureth Sulfate | Texapon N701 | 14.0 | 14.0 | 14.0 | 14.0 |
| Cocamidopropyl Betaine | Tegobetaine CK | 1.6 | 1.6 | 1.6 | 1.6 |
| SeS₂ | | 1.0 | 1.0 | 0.5 | 0.3 |
| Carbomer | Carbopol 980 | 0.60 | 0.60 | 0.60 | 0.60 |
| Silicone Oil | | 2.2 | 2.2 | 2.2 | 2.2 |
| Guar Hydroxypropyltrimonium Chloride | BFG-Jaguar C17 | 0.2 | 0.2 | 0.2 | 0.2 |
| Ethylene Gylcol Distearate (25%) | Euperlan KE4515 | 2.5 | 2.5 | 2.5 | 2.5 |
| Fragrance | | 0.75 | 0.75 | 0.75 | 0.75 |
| pH | | 6.0 | 5.4 | 6.0 | 6.0 |
| Aqua + minors | Water +minors | to 100 | to 100 | to 100 | to 100 |

### Colour stability Test

The samples were incubated at 50°C for 1 week.

All formulations A to D had turned an unacceptable green colour by the end of the test period.

### Example 2 - Addition of Zinc salts provide colour stability

| **INCI name** | **Tradename** | **1 (wt.%)** | **2 (wt.%)** | **3 (wt.%)** | **4 (wt.%)** | **5 (wt.%)** | **6 (wt.%)** |
|---|---|---|---|---|---|---|---|
| Sodium Laureth Sulfate | Texapon N701 | 14.0 | 14.0 | 14.0 | 14.0 | 14.0 | 14.0 |
| Cocamidopropyl Betaine | Tegobetaine CK | 1.6 | 1.6 | 1.6 | 1.6 | 1.6 | 1.6 |
| SeS₂ | | 1.0 | 1.0 | 1.0 | 1.0 | 0.3 | 0.5 |
| Carbomer | Carbopol 980 | 0.60 | 0.60 | 0.60 | 0.60 | 0.60 | 0.60 |
| Silicone Oil | | 2.2 | 2.2 | 2.2 | 2.2 | 2.2 | 2.2 |
| Guar Hydroxypropyltrimonium Chloride | BFG-Jaguar C17 | 0.2 | 0.2 | 0.2 | 0.2 | 0.2 | 0.2 |
| Ethylene Gylcol Distearate (25%) | Euperlan KE4515 | 2.5 | 2.5 | 2.5 | 2.5 | 2.5 | 2.5 |
| Fragrance | | 0.75 | 0.75 | 0.75 | 0.75 | 0.75 | 0.75 |
| Zinc Pyrithione (ZPT) | Zinc Omadine FPS | 0.3 | 0.5 | 1.0 | 1.5 | 1.0 | 1.0 |
| pH | | 5.5 | 5.5 | 5.5 | 5.5 | 5.5 | 5.5 |
| Aqua + minors | Water +minors | to 100 | to 100 | to 100 | to 100 | to 100 | to 100 |

Formulations 1 to 6 were colour stable after incubation at 50°C for 1 week - they did not turn green and remained a light orange colour (faint orange from the 0.5 and 0.3 wt.% SeS₂)

Examples 1 and 2 show that SeS₂ has a colour stability problem that can be solved by including zinc salts (e.g. Zinc Pyrithione (ZPT) in the formulation.

## Claims

1. An antidandruff shampoo comprising:-
a) from 0.5 to 45wt% surfactant;
b) from 0.1 to 2 wt.% of selenium disulfide;
c) from 0.1 to 5 wt.% of zinc pyrithione;
d) from 0.01 to 10 wt.% of a cationic guar hydroxypropyltrimonium chloride polymer; and
e) from 0.01 to 10 wt.% of a silicone.

2. A shampoo according to claim 1, wherein the selenium disulfide is present at a level of from 0.1 to 1.5 wt.%, preferably from 0.1 to 1 wt.%, more preferably 0.1 to 0.5 wt.%.

3. A shampoo according to claim 1 or claim 2, wherein the zinc pyrithione is present at a level of from 0.1 to 3 wt.%, preferably from 0.1 to 2 wt.%.

4. An antidandruff shampoo composition according to any preceding claim, wherein the cationic guar hydroxypropyltrimonium chloride polymer is present at a level of 0.01 to 5 wt.%, preferably from 0.05 to 1 wt.%, more preferably from 0.05 to 0.5 wt.%.

5. An antidandruff shampoo according to any preceding claim, comprising from 0.5 to 20 wt.% alkyl sulfate and/or ethoxylated alkyl sulfate anionic surfactant.

6. An antidandruff shampoo according to any preceding claim, comprising from 0.1 to 10 wt.% of a betaine surfactant, preferably an alkyl amidopropyl betaine.

7. An antidandruff shampoo according to any one of claims 5 to 6, comprising:-
a) from 0.1 to 2 wt.% of selenium disulfide;
b) from 0.1 to 5 wt.% of zinc pyrithione;
c) from 0.01 to 10 wt.% of a cationic guar hydroxypropyltrimonium chloride polymer;
d) from 0.5 to 20 wt.% alkyl sulfate and/or ethoxylated alkyl sulfate anionic surfactant;
e) from 0.1 to 10 wt.% of cocamidopropyl betaine; and,
f) from 0.01 to 10 wt.% of a silicone.

8. An antidandruff shampoo according to any preceding claim, comprising from 0.05 to 5 wt.%, preferably from 0.1 to 2.5 wt.%, more preferably from 0.25 to 1 wt.% of a crosslinked polyacrylate polymer.

9. An antidandruff shampoo according to any preceding claim, comprising from 0.1 to 8 wt.%, more preferably from 0.3 to 5 wt.% of a silicone.

10. An antidandruff shampoo according to any one of claims 1 to 9 for use in the treatment of dandruff.

## Patentansprüche

1. Antischuppenshampoo, Folgendes umfassend:-
a) von 0,5 bis 45 Gew.-% Tensid;
b) von 0,1 bis 2 Gew.-% Selendisulfid;
c) von 0,1 bis 5 Gew.-% Zinkpyrithion;
d) von 0,01 bis 10 Gew.-% eines kationischen Guarhydroxypropyltrimoniumchloridpolymers; und
e) von 0,01 bis 10 Gew.-% eines Silikons.

2. Shampoo nach Anspruch 1, wobei das Selendisulfid in einem Gehalt von 0,1 bis 1,5 Gew.-%, bevorzugt von 0,1 bis 1 Gew.-%, stärker bevorzugt von 0,1 bis 0,5 Gew.-%, vorliegt.

3. Shampoo nach Anspruch 1 oder 2, wobei das Zinkpyrithion in einem Gehalt von 0,1 bis 3 Gew.-%, bevorzugt von 0,1 bis 2 Gew.-%, vorliegt.

4. Antischuppenshampoozusammensetzung nach einem der vorhergehenden Ansprüche, wobei das kationische Guarhydroxypropyltrimoniumchloridpolymer in einem Gehalt von 0,01 bis 5 Gew.-%, bevorzugt von 0,05 bis 1 Gew.-%, stärker bevorzugt von 0,05 bis 0,5 Gew.-%, vorliegt.

5. Antischuppenshampoo nach einem der vorhergehenden Ansprüche, umfassend von 0,5 bis 20 Gew.-% Alkylsulfat und/oder ethoxyliertes anionisches Alkylsulfattensid.

6. Antischuppenshampoo nach einem der vorhergehenden Ansprüche, umfassend von 0,1 bis 10 Gew.-% eines Betaintensids, bevorzugt eines Alkylamidopropylbetains.

7. Antischuppenshampoo nach einem der Ansprüche 5 bis 6, Folgendes umfassend:-
a) von 0,1 bis 2 Gew.-% Selendisulfid;
b) von 0,1 bis 5 Gew.-% Zinkpyrithion;
c) von 0,01 bis 10 Gew.-% eines kationischen Guarhydroxypropyltrimoniumchloridpolymers;
d) von 0,5 bis 20 Gew.-% Alkylsulfat und/oder ethoxyliertes anionisches Alkylsulfattensid;
e) von 0,1 bis 10 Gew.-% Cocamidopropylbetain; und
f) von 0,01 bis 10 Gew.-% eines Silikons.

8. Antischuppenshampoo nach einem der vorhergehenden Ansprüche, umfassend von 0,05 bis 5 Gew.-%, bevorzugt von 0,1 bis 2,5 Gew.-%, stärker bevorzugt von 0,25 bis 1 Gew.-%, eines vernetzten Polyacrylatpolymers.

9. Antischuppenshampoo nach einem der vorhergehenden Ansprüche, umfassend von 0,1 bis 8 Gew.-%, stärker bevorzugt von 0,3 bis 5 Gew.-%, eines Silikons.

10. Antischuppenshampoo nach einem der Ansprüche 1 bis 9 zur Verwendung bei der Behandlung von Schuppen.

## Revendications

1. Shampooing antipelliculaire comprenant : -
a) de 0,5 à 45 % en poids de tensioactif ;
b) de 0,1 à 2 % en poids de disulfure de sélénium ;
c) de 0,1 à 5 % en poids de pyrithione de zinc ;
d) de 0,01 à 10 % en poids d'un polymère de chlorure d'hydroxypropyltrimonium de guar cationique ; et
e) de 0,01 à 10 % en poids d'un silicone.

2. Shampooing selon la revendication 1, dans lequel le disulfure de sélénium est présent à un niveau de 0,1 à 1,5 % en poids, de préférence de 0,1 à 1 % en poids, plus préférablement de 0,1 à 0,5 % en poids.

3. Shampooing selon la revendication 1 ou la revendication 2, dans lequel la pyrithione de zinc est présente à un niveau de 0,1 à 3 % en poids, de préférence de 0,1 à 2 % en poids.

4. Composition de shampooing antipelliculaire selon l'une quelconque des revendications précédentes, dans laquelle le polymère de chlorure d'hydroxypropyltrimonium de guar cationique est présent à un niveau de 0,01 à 5 % en poids, de préférence de 0,05 à 1 % en poids, plus préférablement de 0,05 à 0,5 % en poids.

5. Shampooing antipelliculaire selon l'une quelconque des revendications précédentes, comprenant de 0,5 à 20 % en poids d'alkylsulfate et/ou de tensioactif anionique alkylsulfate éthoxylé.

6. Shampooing antipelliculaire selon l'une quelconque des revendications précédentes, comprenant de 0,1 à 10 % en poids d'un tensioactif bétaïne, de préférence une alkylamidopropylbétaïne.

7. Shampooing antipelliculaire selon l'une quelconque des revendications 5 à 6, comprenant : -
a) de 0,1 à 2 % en poids de disulfure de sélénium ;
b) de 0,1 à 5 % en poids de pyrithione de zinc ;
c) de 0,01 à 10% en poids d'un polymère de chlorure d'hydroxypropyltrimonium de guar cationique ;
d) de 0,5 à 20 % en poids d'alkylsulfate et/ou de tensioactif anionique alkylsulfate éthoxylé ;
e) de 0,1 à 10 % en poids de cocamidopropylbétaïne ; et,
f) de 0,01 à 10 % en poids d'un silicone.

8. Shampooing antipelliculaire selon l'une quelconque des revendications précédentes, comprenant de 0,05 à 5 % en poids, de préférence de 0,1 à 2,5 % en poids, plus préférablement de 0,25 à 1 % en poids d'un polymère de polyacrylate réticulé.

9. Shampooing antipelliculaire selon l'une quelconque des revendications précédentes, comprenant de 0,1 à 8 % en poids, plus préférablement de 0,3 à 5 % en poids d'un silicone.

10. Shampooing antipelliculaire selon l'une quelconque des revendications 1 à 9 destiné à être utilisé dans le traitement des pellicules.
